# EUROPÄISCHE PATENTSCHRIFT

(11) **EP 2 413 873 B1**
(45) Veröffentlichungstag und Bekanntmachung des Hinweises auf die Patenterteilung: **25.02.2015**
(21) Anmeldenummer: 10800699.0
(22) Anmeldetag: 09.12.2010
(51) Int. Cl.: A61G 3/00

(54) **VORRICHTUNG FÜR DEN TRANSPORT EINER NICHT GEHFÄHIGEN PERSON IN LIEGENDEM ZUSTAND**
DEVICE FOR TRANSPORTING A NON-AMBULANT PERSON IN A LYING POSITION
DISPOSITIF POUR LE TRANSPORT EN POSITION ALLONGÉE D'UNE PERSONNE INCAPABLE DE MARCHER

(30) Priorität: 09.12.2009 DE 102009057372
(43) Veröffentlichungstag der Anmeldung: 08.02.2012
(73) Patentinhaber: Moeck, Lidia, 22607 Hamburg (DE)
(72) Erfinder: Moeck, Lidia, 22607 Hamburg (DE)
(74) Vertreter: RGTH
(86) Internationale Anmeldenummer: PCT/EP2010/007493
(87) Internationale Veröffentlichungsnummer: WO 2011/069662

(56) Entgegenhaltungen:
- CA-A1- 2 388 345
- CH-A- 339 699
- GB-A- 716 746
- JP-A- 62 120 214
- US-A- 5 716 269
- US-A1- 2006 272 231

## Beschreibung

Die Erfindung betrifft einen Rettungswagen mit einem Ausgang einer Klimaanlage und/oder einen Ausgang einer Heizungsanlage zum Kühlen bzw. Wärmen eines Patientenraumes.

### Stand der Technik

Übliche Rettungswagen bzw. Krankentransportfahrzeuge sind mit einem Patientenraum versehen, in dem sich während des Transports der Patient sowie die Ärzte sowie die betreuenden Sanitäter befinden und der zu diesem Zweck mit medizinischen Gerätschaften ausgestattet ist. Zudem ist der Patientenraum mit einem Ambulanztisch ausgestattet, auf dem der Patient mitsamt einer Fahrtrage, auf der er zuvor zum Rettungswagen befördert wurde, liegt. Hierzu weist der Ambulanztisch üblicherweise eine ausziehbare Tragenaufnahme und eine geeignete Kinematik auf, mittels der die Tragenaufnahme um eine horizontale Querachse kippbar ist, so dass das nach dem Ausziehen aus dem Heck des Rettungswagens herausragende Beladeende der Trageaufnahme absenkbar ist, um das Aufschieben der Trage mitsamt Patient auf die Trageaufnahme zu erleichtern. Nach dem Aufschieben und Fixieren der Trage auf der Trageaufnahme wird die Trageaufnahme mittels der Kinematik zurück in eine horizontale Lage geschwenkt und anschließend mitsamt der Trage per Hand in ihre Transportstellung eingefahren. Rettungswagen verfügen zudem in den Patientenräumen über Ausgänge für die wageneigenen Klima- und Heizungsanlagen zum Kühlen bzw. Wärmen des Patientenraumes, d. h. zur Aufrechterhaltung einer bestimmten Raumkühlung bzw. Raumtemperatur.

Die Kühlung und Erwärmung mittels der wageneigenen Klima- und Heizungsanlagen stellt jedoch kein ausreichendes Instrumentarium dar, die Kühlung von Patienten, beispielsweise nach einer Reanimation, in dem Patientenraum zu gewährleisten oder dem Patienten als homöothermem Lebewesen die nötige Wärmezufuhr zukommen zu lassen, um im Bedarfsfall eine konstante Körpertemperatur zur Sicherstellung überlebenswichtiger Funktionen zu gewährleisten.

Bisherige technische Möglichkeiten wie die Zuführung von konduktiver Wärme mittels Heizdecken, die bspw. auf Kohlefaser basieren, bieten keine Abhilfe, da diese den Nachteil einer vorgeschriebenen Temperaturbegrenzung haben. Zudem bieten derartige Heizdecken keine Druckentlastung von unten, so dass das periphere Gefäßsystem teilweise die zugeführte Wärme nicht schnell genug in den Körper abtransportieren kann. Die technische Möglichkeit einer konduktiven Kühlung des Patienten ist auch nur begrenzt, da diese nur mittels partiell applizierten Kühlpacks möglich ist.

Aus der CH 339 699 A ist eine mobile Klinik, die aus mehreren in der Parkstellung aneinandergereihten Fahrzeugen besteht, bekannt. Es werden Abdeckelemente und Rohrleitungen verwendet, die in Zusammenhang mit einer Klimaanlage eines Klinikwagens stehen.

Die Druckschrift GB 716 746 A zeigt und beschreibt eine Wärme- bzw. Kühldecke für den Hausgebrauch mit einem Anschlussschlauch 6. Der Schlauch ist mit einem Kasten zur z.B. Wärmeerzeugung verbunden.

Die US 5,716,269 offenbart einen Rettungswagen mit einer Klimaanlage.

### Darstellung der Erfindung: Aufgabe, Lösung, Vorteile

Unter Würdigung des umrissenen Standes der Technik bei Rettungswagen der eingangs genannten Art liegt daher der vorliegenden Erfindung die Aufgabe zugrunde, einen Rettungswagen der eingangs genannten Art derart weiterzuentwickeln, dass auch im Patientenraum des Rettungswagens eine lebenswichtige Wärme und Kühlversorgung des Patienten gegeben ist.

Diese Aufgabe wird durch einen Rettungswagen mit den im Anspruch 1 angegebenen Merkmalen gelöst. Vorteilhafte Ausgestaltungen und zweckmäßige Weiterbildungen der vorliegenden Erfindung sind in den Unteransprüchen gekennzeichnet.

Gemäß der Erfindung ist der Ausgang mit einer eine Öffnung aufweisenden Abdeckplatte versehen, wobei der Öffnung eine Leitung zur Förderung von Warmluft und/oder Kaltluft zugeordnet ist.

Kernidee der Erfindung ist es, über die rettungswageneigenen Ausgänge für eine Klimaanlage und/oder für eine Heizungsanlage mittels der mit einer Leitung versehenen Abdeckplatte, deren Öffnung vorzugsweise zentriert ist, Warm- und Kühlluft zu einer Wärme- bzw. Kühldecke zu leiten.

Der Vorteil der Erfindung ist darin zu sehen, dass ein Patient in einem Patientenraum eines Rettungswagens sehr schnell mit Warm- bzw. Kaltluft versorgt werden kann, sobald die Wärmedecke bzw. Kältedecke über einen Verbindungsschlauch mit der Leitung der Abdeckplatte verbunden wird. Hierdurch kann womöglich lebensrettende Zeit gewonnen werden. Insbesondere kann vorteilhafterweise von Wärmedecken Gebrauch gemacht werden, bei denen eine konvektive Wärmung von unten mit integrierter Druckentlastung erfolgt. Bei der Wärmedecke kann es sich um eine herkömmliche Wärmedecke handeln, die als Wärmeunterlage bei Operationen am Körper zum Einsatz kommen kann. Diese vorbekannte Wärmedecke weist einen Anschluss für den Verbindungsschlauch auf, über den Warmluft aus der Leitung der Abdeckplatte zugeführt werden kann.

Die Warmluft verteilt sich sodann in der als Zwischenschicht ausgebildeten Zwischenlage, die in der insgesamt dreilagig ausgebildeten Wärmedecke zwischen der als Innenschicht ausgebildeten, dem Körper zugewandten Innenlage und der als Außenschicht ausgebildeten, vom Körper abgewandten Außenlage, die auch als Sperrschicht bezeichnet wird, eingebracht ist (die Zwischenlage oder Zwischenschicht wird also durch die körperzugewandte Innenlage oder Innenschicht sowie durch die körperabgewandte Außenlage oder Außenschicht (Sperrschicht) begrenzt).

Durch das Vorsehen der Zwischenlage kann die von der Warmluftquelle zur Verfügung gestellte Warmluft in jedem Falle kontinuierlich unter dem zu wärmenden (oder zu kühlenden) Körper hindurch geführt werden, so dass die auf der Wärmedecke auflagernden Körperteile unter jeglichen Bedingungen zuverlässig gewärmt werden.

Hierzu ist der innenliegende Raum oder "Innenraum" der Wärmedecke mit einem Zwischenlagenmaterial aus einem aus Chemiefasern hergestellten abstandhaltenden, textilen Flächengebilde, beispielsweise in Gestalt eines Polyesterabstandsgewirkes, gefüllt; dieses Material der Zwischenlage ist in erfindungswesentlicher Weise einerseits so fest, stabil und starr, dass der Durchlass von Warmluft sichergestellt ist, andererseits weist eben dieses Material der Zwischenlage einen Elastizitätsgrad auf, der ein druckentlastendes Lagern von narkotisierten Patientinnen und Patienten, insbesondere von Babys und von Kleinkindern, gewährleistet.

Des Weiteren werden sowohl die Innenlage oder Innenschicht als auch die Außenlage oder Außenschicht durch das Anordnen der Zwischenlage oder Zwischenschicht nicht so sehr zusammengedrückt, dass ein Durchlassen, ein Durchleiten, ein Durchströmen und/oder ein Transportieren der Warmluft nicht mehr möglich ist.

Die Außenlage der Wärmedecke ist hierbei luftdicht und luftundurchlässig ausgebildet, wozu die Außenlage im Wesentlichen aus Textilmaterial besteht. Im Gegensatz dazu ist die Innenlage der Wärmedecke insofern luftdurchlässig ausgebildet, als diese Innenlage aus Mikrofasermaterial besteht und feinste, insbesondere porenartig ausgebildete Öffnungen zum Ausströmen der zugeführten Warmluft aufweist.

Die vorgenannten technischen Maßnahmen führen dazu, dass die Warmluft/Kaltluft im Wesentlichen ausschließlich an der Innenlage austreten kann, die beim Einsatz der Wärmedecke dem Körper einer Patientin oder eines Patienten zugewandt ist. Auf diese Weise kann die als Wärmeunterlage fungierende Wärmedecke gleichzeitig für eine Isolierung gegen Wärmeverlust und zusätzlich für eine aktive Wärmezufuhr sorgen.

Zudem können im Rahmen der Erfindung beispielsweise auch vorbekannte Wärmedecken Verwendung finden, bei denen konduktive Wärme dem Patienten unter- und oberhalb des Patienten zugeführt wird.

Die Leitung ist an dem Ausgang mit der Abdeckplatte fest oder lösbar verbunden. Hierzu sieht eine praktikable Variante der Erfindung vor, dass die Leitung z. B. über einen Bajonettverschluss an der Öffnung angeschlossen ist. Die Leitung kann als Rohr oder in flexibler Form als Schlauch ausgebildet sein.

Eine vorteilhafte Ausgestaltung der Erfindung sieht vor, dass die Abdeckplatte auf der dem Ausgang zugewandten Seite eine Dichtlippe oder eine Dichtungsschaumstofffläche aufweist. Die Dichtlippe und die Dichtungsschaumstofffläche kann dabei die gesamte Seite mit Ausnahme der Öffnung bedecken oder im Randbereich der Abdeckplattenseite angeordnet sein. Dadurch, dass die Abdeckplatte mit diesen Dichtlippen bzw. Dichtungsschaumstoffflächen versehen ist, ist gewährleistet, dass Warmluft bzw. Kühlluft während des Austritts aus dem Ausgang nicht entweicht.

Vorteilhafterweise ist in der Leitung eine Bimetallfeder angeordnet. Eine Bitmetallfeder besteht aus aufeinander plattierten Flächen unterschiedlicher Wärmeausdehnung. Wegen der temperaturabhängigen Formänderung der Bimetallfeder lässt sich diese zu Steuerungszwecken einer in die Leitung integrierten Klappe verwenden. Dazu ist zunächst in der Leitung vorzugsweise eine Öffnung und eine bewegliche Vorrichtung zum Schließen der Öffnung vorgesehen. Die Vorrichtung und die Bimetallfeder sind dabei derart miteinander gekoppelt, dass sich die Vorrichtung, die vorzugsweise als Klappe vorliegt, schrittweise der temperaturinduzierten Verbiegung eines Streifens öffnet. Auf diese Weise gelangt kühle Zuluft in das Innere der Leitung und durch den hierdurch entstehenden Unterdruck wird eine notwendige Temperaturbegrenzung bzw. Temperaturbegrenzung bzw. Temperaturstabilität, die bei ca. 43° C liegt, erreicht. Als praktikable Variante sieht die Erfindung auch vor, dass in der Leitung ein Temperatursensor angeordnet ist, der vorzugsweise über eine batteriebetriebene Soft- und Hardware die Vorrichtung steuert.

Zudem sieht die Erfindung einen Rettungswagen mit einer Abdeckplatte mit einer Öffnung vor, wobei die Abdeckplatte mit einer Öffnung versehen ist, der eine Leitung zur Förderung von Warmluft und/oder Kaltluft zugeordnet ist.

### Kurze Beschreibung der Zeichnungen

Weitere Ausgestaltungen, Merkmale und Vorteile der vorliegenden Erfindung werden nachstehend anhand der durch die Figuren 1 bis 9 veranschaulichten Ausführungsbeispiele näher erläutert. Es zeigen in schematischer Darstellung:
Fig. 1 eine seitliche Ansicht eines Rettungswagens gemäß der Erfindung;
Fig. 2 in perspektivischer Ansicht einen Ausschnitt eines mit einem Ausgang versehenen Patientenraumes in einem Rettungswagen;
Fig. 3 in perspektivischer Ansicht eine Abdeckplatte gemäß der Erfindung;
Fig. 4 in perspektivischer Ansicht die Leitung einer Abdeckplatte, wobei die Leitung eine Öffnung und eine Vorrichtung zum Schließen der Öffnung sowie eine Bimetallfeder aufweist;
Fig. 5 in perspektivischer Ansicht eine mit einer Befestigungsvorrichtung versehene erfindungsgemäße Abdeckplatte sowie ein Gitter eines Ausgangs in einem Rettungswagen;
Fig. 6 eine Draufsicht auf eine Abdeckplatte mit einem Bajonettverschluss gemäß der Erfindung;
Fig. 7 die Rückseite der Abdeckplatte aus Fig. 6;
Fig. 8 eine Draufsicht eines Heizungsausgangs mit Temperaturregelung;
Fig. 9 in perspektivischer Ansicht eine Trage mit einer Wärmedecke;
Fig. 10 die Trage aus Fig. 9 mit einem auf der Trage liegenden Patienten;
Fig. 11 eine Draufsicht auf eine Wärmedecke mit Griffen; und
Fig. 12 Schnittansichten der Wärmedecke aus Fig. 11 mit integrierten Griffen entlang der Schnittlinie 2-II aus Fig. 11.

### Bevorzugte Ausführungsform der Erfindung

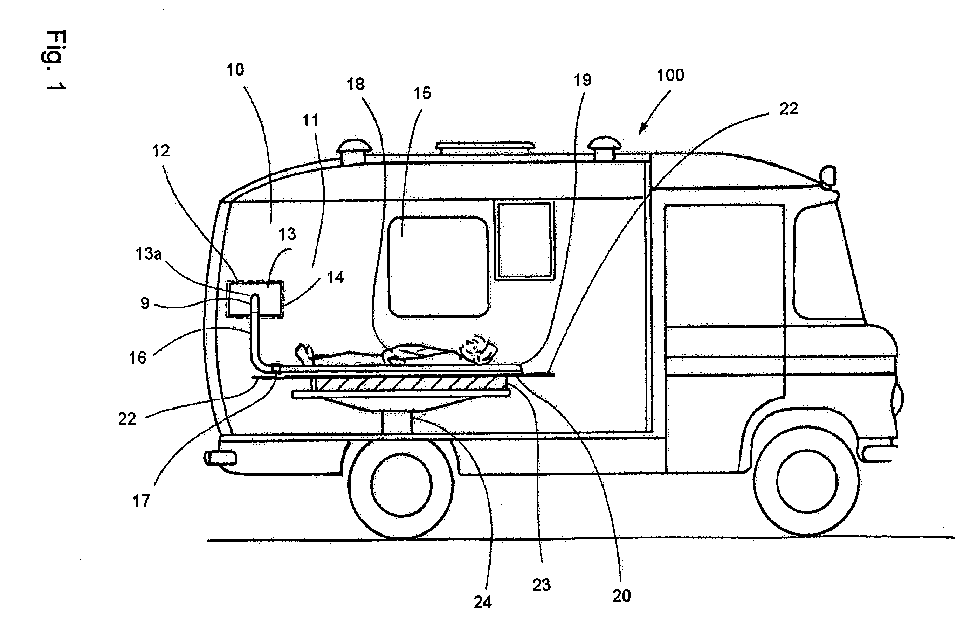

Aus Fig. 1 geht ein erfindungsgemäßer Rettungswagen 100 hervor, der einen Fahrerraum und einen Patientenraum 10, in dem während des Krankentransportes die Operationen, bzw. lebensrettende Maßnahmen durchgeführt werden und in dem sich neben dem zu behandelnden Patienten 18 die behandelnden Ärzte bzw. Sanitäter befinden. Der mit einem Fenster 15 versehene quaderförmige Patientenraum 10 weist die zur Versorgung des Patienten 18 notwendigen medizinischen Gerätschaften auf. Hierzu zählt der Ambulanztisch 24, der zur Aufnahme der Trage 20 mitsamt dem darauf liegenden Patienten 18 dient. Der Ambulanztisch 24 ist fest auf dem Boden des Patientenraumes 10 angeordnet und weist einen Untertisch auf, d. h. eine plattenförmige Tragenaufnahme 23, die mittels einer Betätigungseinrichtung vorzugsweise höhenverstellbar ist. Bei der Trage 20 kann es sich auch um eine Fahrtrage mit einem zusammengeklappten Fahrgestell handeln, welches auf der Tragenaufnahme aufliegt.

Auf der Liegefläche 22 der Trage 20 und unterhalb des Patienten 18 befindet sich die Wärmedecke 19, die mit der Trage 20 fest oder lösbar verbunden ist. In dem in Fig. 1 gezeigten Ausführungsbeispiel eines Rettungswagens 100 ist der Patient 18 samt der Trage 20 zuvor zum Rettungswagen 100 befördert worden, um dann mittels der ausziehbaren Tragenaufnahme 23 und einer geeigneten Kinematik in den Patientenraum 10 zu gelangen. Hierzu ist vorgesehen, dass die Trageaufnahme 23 um eine horizontale Querachse kippbar ist, so dass das nach dem Ausziehen aus dem Rettungswagen 100 herausragende Beladeende der Trageaufnahme 23 abknickbar ist, um das Aufschieben der Trage 20 mitsamt Patienten 18 auf die Trageaufnahme 23 zu erleichtern. Nach dem Aufschieben und Fixieren der Trage 20 auf der Trageaufnahme 23 wird die Trageaufnahme 23 dann mittels der Kinematik zurück in eine horizontale Lage geschwenkt. Eine praktikable Variante der Erfindung kann derart sein, dass der Patient 18 samt Trage 20 direkt von den Ärzten bzw. Sanitätern in den Patientenraum 10 getragen wird, um die Trage 20 direkt auf die Tragenaufnahme 23 zu legen.

Der Rettungswagen 100 verfügt zudem über eine Heizungsanlage mit einem Ausgang 14, aus dem die Warmluft gelangt. Zwischen Ausgang 14 der Heizungsanlage und Wärmedecke 19 ist der Verbindungsschlauch 16 angeordnet, der direkt mit der Leitung 9 der Abdeckplatte 13 verbunden ist. Die Leitung 9 ist wiederum der zentrierten Öffnung 13a der Abdeckplatte 13 zugeordnet und zwar derart, dass die Leitung 9, beispielsweise über einen Bajonettverschluss an der Öffnung 13a angebracht ist. Auf diese Weise ist gewährleistet, dass die aus dem Ausgang 14 der Heizungsanlage ausströmende Warmluft über die Leitung 9 in den Verbindungsschlauch 16 gelangt, der wiederum mit dem Anschluss 17 der Wärmedecke 19 verbunden ist, so dass die Warmluft aus der rettungswageneigenen Heizungsanlage der Wärmedecke 19 direkt zugeführt werden kann. Der Randbereich der Oberfläche der Abdeckplatte 13 ist in Kontakt mit der den Ausgang 14 umgebenden Wand 11 des Patientenraumes 10, so dass die Abdeckplatte 13 problemlos an der Wand 11 lösbar oder unlösbar montierbar ist. Alternativ kann - sofern ein Heizungsgitter 12 am Ausgang 14 der Heizungsanlage vorgesehen ist - die Abdeckplatte 13 direkt mittels einer Befestigungsvorrichtung mit dem Heizungsgitter 12 verbunden werden.

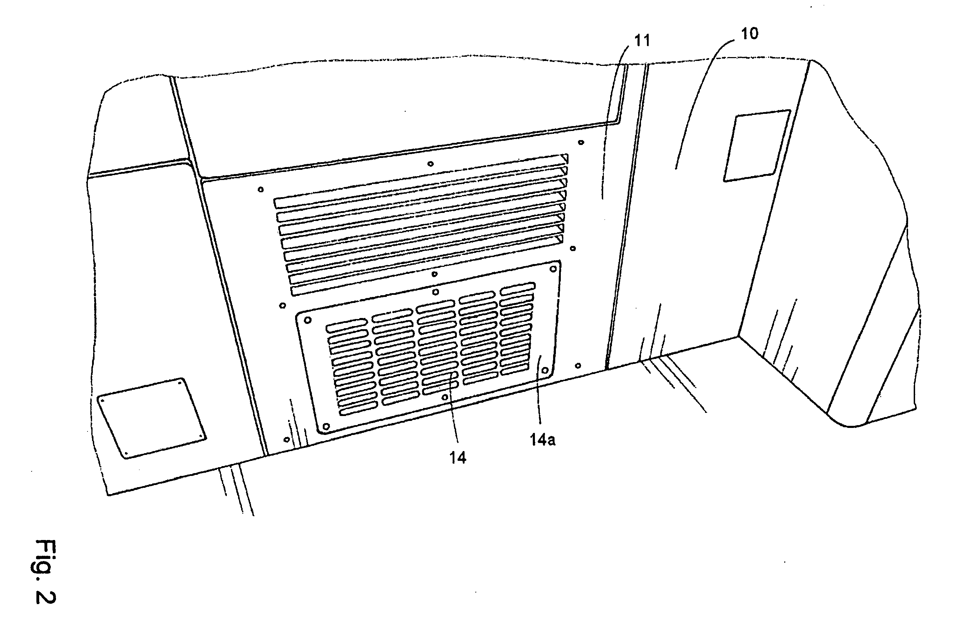

Fig. 2 veranschaulicht einen mit einem Gitter 14a versehenen Ausgang 14 in einem Patientenraum 10 eines herkömmlichen Rettungswagens. Der Ausgang 14 ist dabei integraler Bestandteil der Wand 11 des Patientenraumes 10, an dem die in Fig. 1 gezeigte Abdeckplatte 13 montiert werden kann.

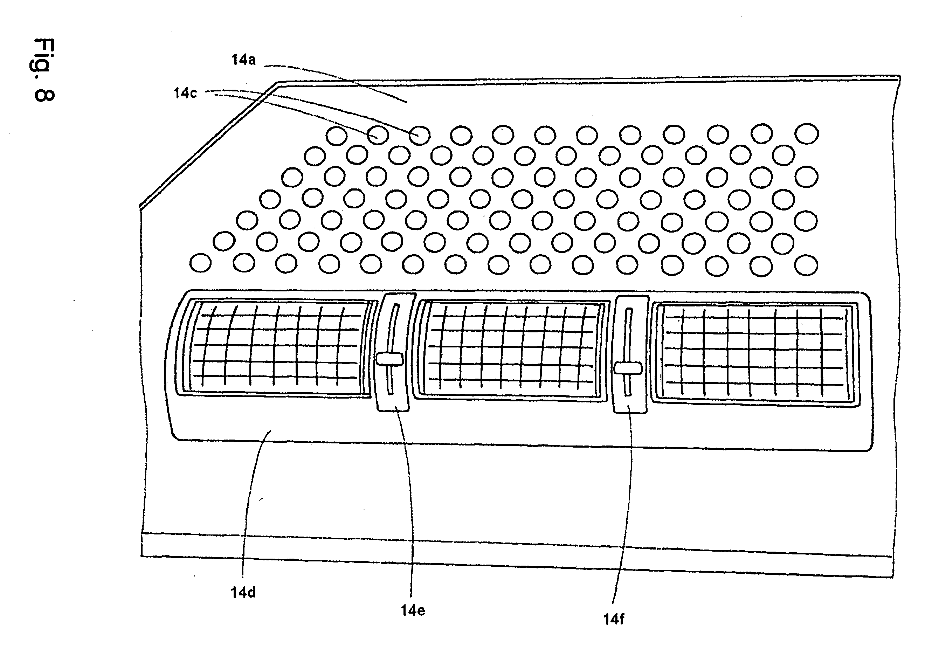

Zudem kann die aus einem Gitter 14c eines Ausgangs 14 in einem Patientenraum eines Rettungswagens ausströmende Warmluft über Temperatur- bzw. Warmluftregler 14e, 14f einer hierfür vorgesehenen Steuerung 14d geregelt werden (Fig. 8).

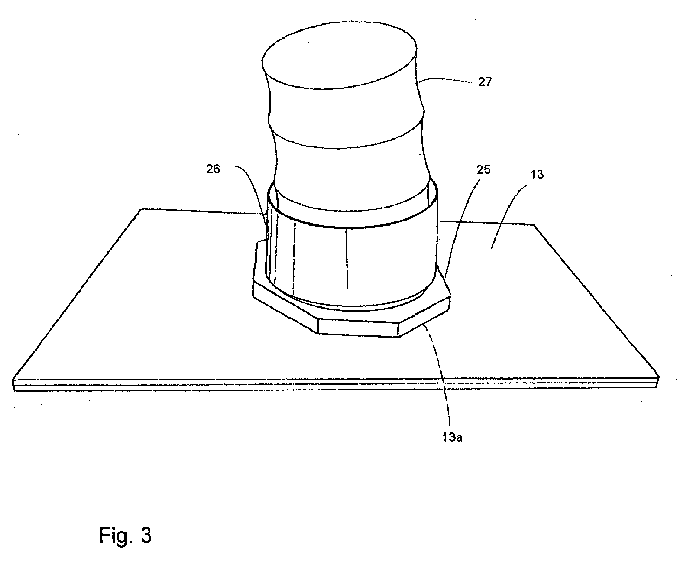

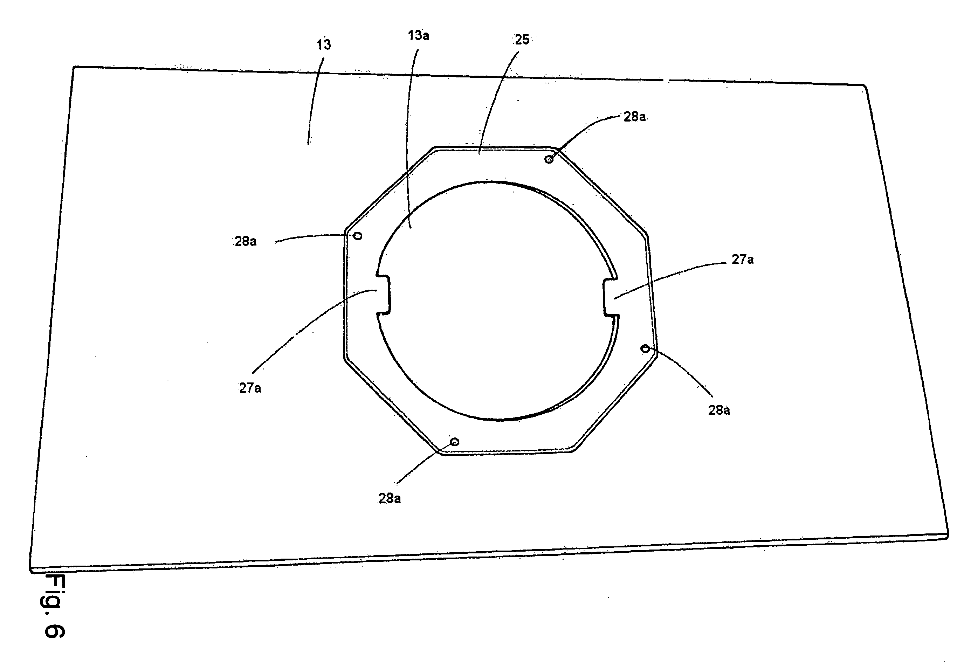

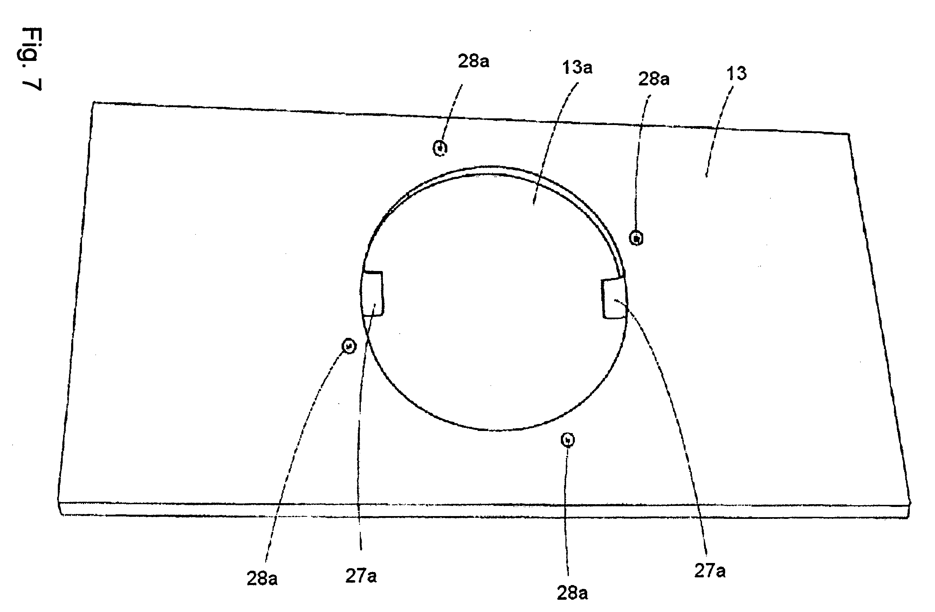

Die Platte 13 gemäß der Erfindung geht aus der Fig. 3 hervor. Die Platte 13 weist eine Öffnung 13a auf. Die in Fig. 3 lediglich angedeutete Öffnung 13a ist kreisförmig und z. B. ungefähr mittig der Platte 13 ausgebildet. Die Platte 13 ist von rechteckiger Gestalt und aus Metall oder Kunststoff. In die Öffnung 13a ist die Leitung 26, die als Schlauch oder Rohr vorliegen kann, über einen Bajonettverschluss 25 eingesetzt und mit der Platte 13 fest verbunden. Die Leitung 26 hat dabei einen Durchmesser, der dem Durchmesser der Öffnung 13a entspricht. In die Leitung 26 ist der Verbindungsschlauch 27, der in Fig. 3 die Form eines Spiralschlauches aufweist, eingesetzt. Um die Verbindung zwischen Leitung 26 und Verbindungsschlauch 27 herzustellen, kann im Rahmen der Erfindung vorgesehen sein, dass die Leitung 26 und der Verbindungsschlauch 27 miteinander verklebt sind. Bei der in Fig. 3 gezeigten Ausführungsform erfolgt erwähntermaßen die Verbindung zwischen Leitung 26 und Platte 13 über einen Bajonettverschluss 25. Bei dem Bajonettverschluss 25 handelt es sich um eine hinlänglich bekannte Vorrichtung zum leicht lösbaren Verbinden zweier Teile und zwar im Rahmen der vorliegenden Erfindung zum Verbinden der Platte 13 und der Leitung 26. Hierzu besitzt die Leitung einen in Fig. 3 nicht gezeigten Schlitz, an dessen Ende vorzugsweise rechtwinklig ein kurzer Querschlitz ansetzt. Der andere Teil des Bajonettverschlusses 25, der den Rand der Öffnung 13a fest umgibt, besitzt dagegen Knöpfe (sog. Zähne) 27a, die in den Querschnitt eingeführt werden und somit die feste Verbindung bewirken. Der Teil des Bajonettverschlusses 25, der sich in der Öffnung 13a befindet, ist mittels Verbindungsmitteln 28a, die beispielsweise in Gestalt von Schrauben vorliegen können, mit der Platte verbunden (Fig. 6, 7).

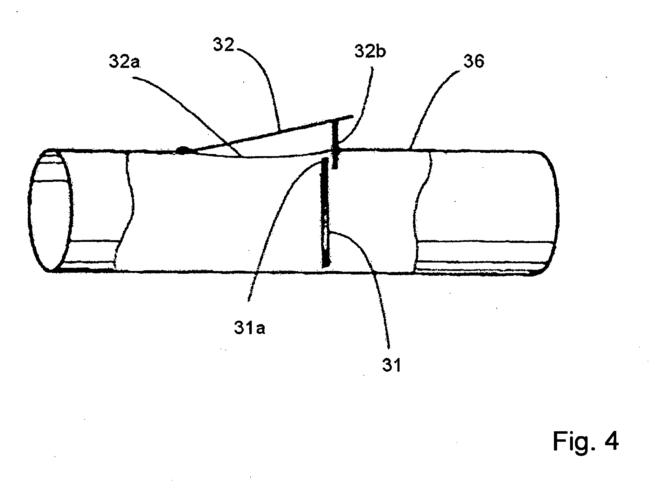

Fig. 4 zeigt den Ausschnitt einer Leitung 36 einer erfindungsgemäßen Abdeckplatte, in die eine Bimetallfeder 31 integriert ist. Zudem verfügt die Leitung 36 über eine Öffnung 32a, die mittels der Vorrichtung 32 geschlossen werden kann. Die Vorrichtung 32 erfüllt somit die Funktion einer Klappe. Die Bimetallfeder 31, die im Wesentlichen aus Metallstreifen aus zwei Schichten unterschiedlicher Materialien, die miteinander stoffschlüssig oder formschlüssig verbunden ist, wird in der in Fig. 4 gezeigten Ausführungsform einer Leitung 36 zu Steuerungszwecken verwendet. Hierzu kann im Rahmen der Erfindung vorgesehen sein, dass durch eine Temperaturveränderung im Inneren der Leitung 36 eine Verbiegung der Metallstreifen der Bitmetallfeder 31 stattfindet, wodurch das Ende 31a der Bimetallfeder 31 seine Position verändert und das Kontaktteil 32b der Vorrichtung 32 kontaktiert und somit die Vorrichtung 32 bewegt, so dass eine Luftzufuhr von außen in die Leitung 36 gelangen kann.

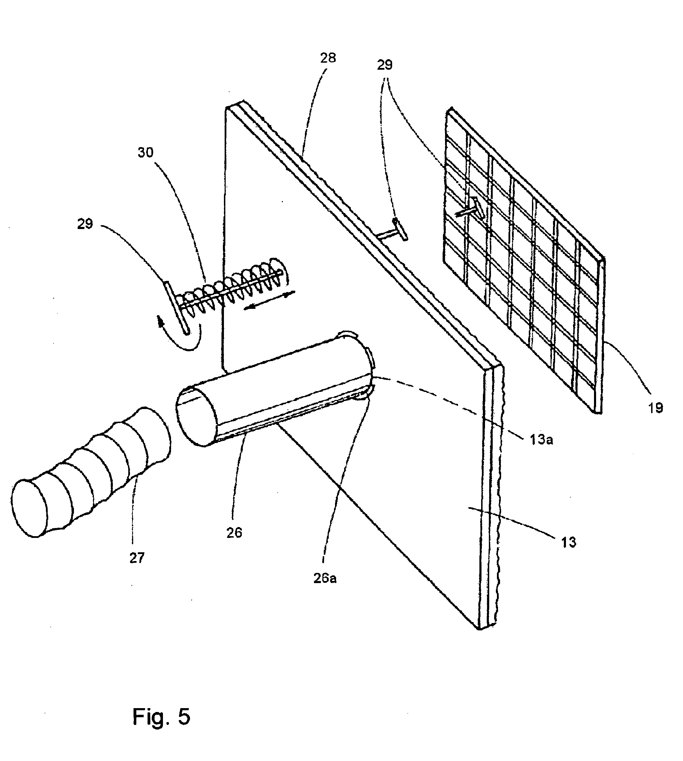

Eine bevorzugte konstruktive Maßnahme zur Befestigung der erfindungsgemäßen Platte 13 an einem Gitter 19 eines Ausgangs in einem Patientenraum 10 eines Rettungswagens geht aus Fig. 5 hervor. Hierzu kann im Rahmen der Erfindung vorgesehen sein, dass an der Platte 13 T-förmige und mit einer Feder 30 versehene Halteklammern als Befestigungsvorrichtungen 29 montiert sind, die in die Gitterlöcher des Gitters 19 geführt werden und sodann derart gedreht werden können, dass sie in das Gitter 19 greifen und somit eine feste Anbringung der Platte 13 an dem Gitter 19 gegeben ist. Die Platte 13 ist zudem auf der dem Gitter 19 zugewandten Seite mit einer Dichtlippe 28 versehen. Die Dichtlippe 28 kann mit der Platte 13 verklebt sein und die gesamte Fläche mit Ausnahme der Öffnung 13a bedecken. Ein Bajonettverschluss 26 a sorgt für eine feste Anbringung der Leitung 26 an der in Fig. 5 nur angedeuteten Öffnung 13a der Platte 13. Eine weitere Verbindung kann zudem zwischen der Leitung 26 und dem zu einer Wärmedecke 19 führenden Verbindungsschlauch 27 erfolgen.

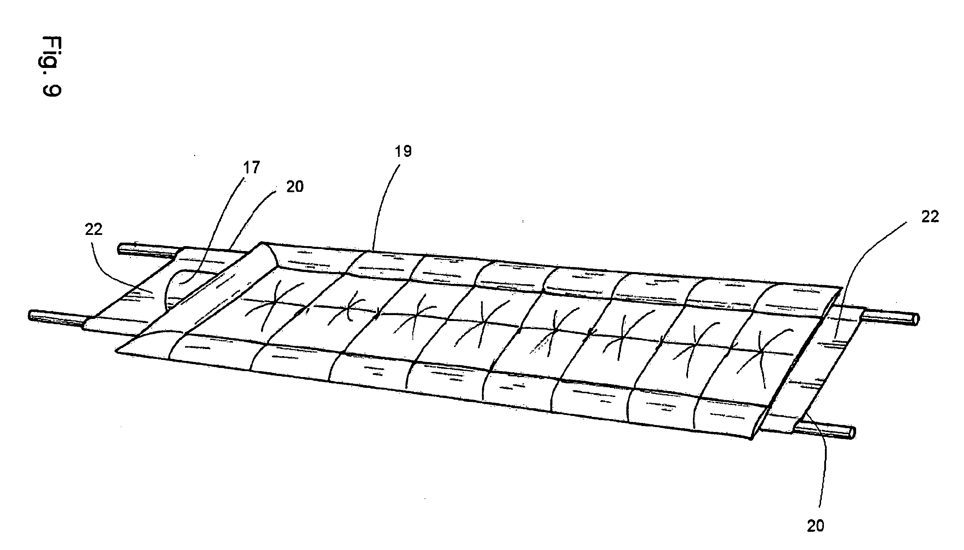

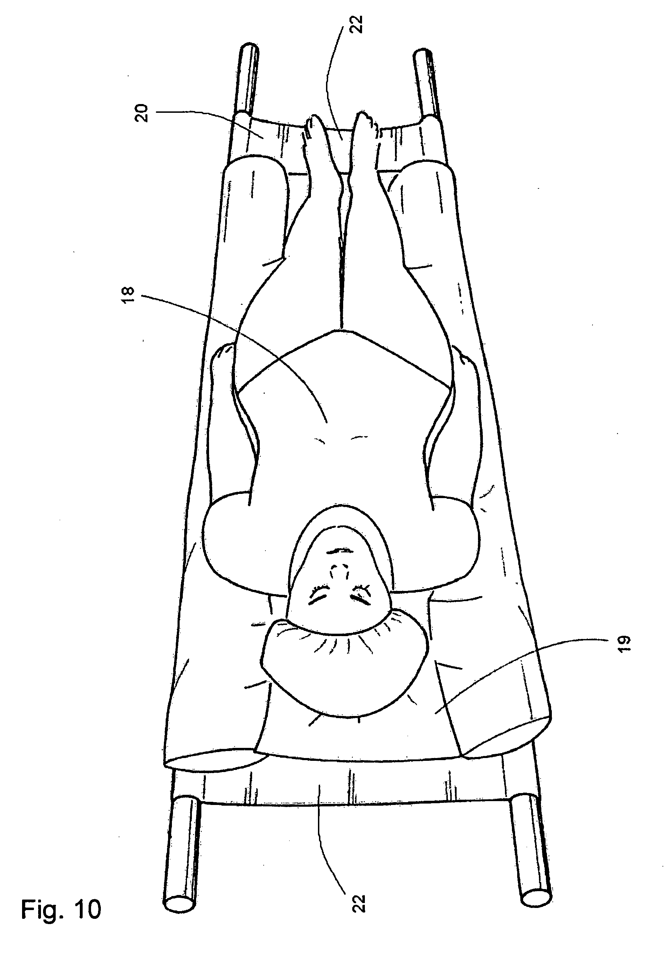

Aus den Fig. 9 und 10 geht eine Ausführungsform einer Trage 20 mit Wärmedecke 19 hervor, die auch in einem Patientenraum des in Fig. 1 gezeigten erfindungsgemäßen Rettungswagen 100 Verwendung finden kann. Die Trage 20 sowie die Wärmedecke 19 sind dabei beispielhaft, da von Tragen und Wärmedecken unterschiedlicher Ausgestaltungen Gebrauch gemacht werden kann. Beispielsweise kann für den Transport eines Patienten 18 von vornherein eine Fahrtrage Verwendung finden. Auch können unterschiedliche Wärmedecken, wie sie bspw. von der Firma Moeck und Moeck hergestellt und vertrieben werden, verwendet werden. Insbesondere sind auch Einwegpapierwärmedecken geeignet. Die Trage 20 zeichnet sich insbesondere dadurch aus, dass die Wärmedecke 19 auf der Liegefläche 22 der Trage 20 angeordnet ist. Die Wärmedecke 19 liegt auf der Liegefläche 22 auf und steht mit ihrer Außenlage in unmittelbarem Kontakt zur Liegefläche 22.

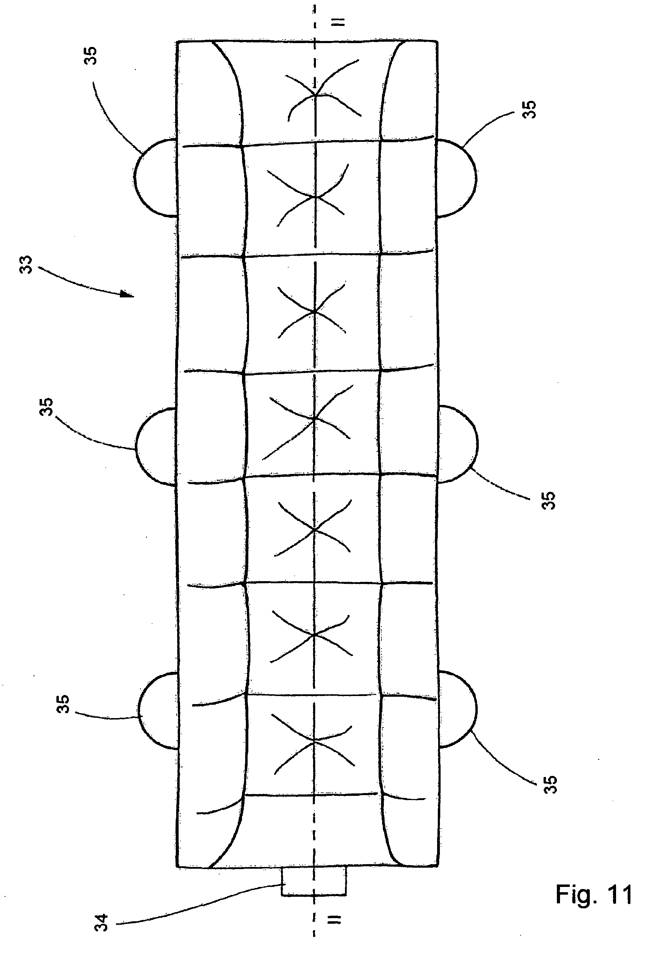

Die Liegefläche 22 ist in den in den Fig. 9 und 11 gezeigten Ausführungsformen fest mit der Wärmedecke 19 verbunden. Die feste Verbindung kann bspw. in Gestalt einer Verklebung gegeben sein. Eine kühlere Außenlage der Wärmedecke 19 hat dabei den Vorteil, dass diese Außenfläche fester auf der Liegefläche 22 fixiert ist, da sich bspw. Klebestreifen bei niedrigen Temperaturen weniger leicht lösen. Eine weitere Variante einer festen Verbindung zwischen Wärmedecke 19 und Liegefläche 22 der Trage 20 ist eine Vernähung. Im Rahmen der Erfindung ist es jedoch auch möglich, dass Liegefläche 22 und die Wärmedecke 19, bspw. mittels eines Knopfverschlusses, lösbar miteinander verbunden sind.

Fig. 11 zeigt eine weitere Wärmedecke 33, die im Rahmen der Erfindung Verwendung finden kann. Die Wärmedecke 33/Unterlage weist Handgriffe 35 auf, die seitlich der Wärmedecke 33 angeordnet sind. Die Handgriffe 35 können unterschiedlich ausgestaltet sein. In der in Fig. 11 gezeigten Ausführungsform der Wärmedecke 33 liegen die Handgriffe 35 in Form von Lederbändern oder Stoffbändern vor und sind halbkreisförmig gestaltet. Die Handgriffe 35 sind seitlich der Wärmedecke 33 mit dieser fest verbunden. Hierzu kommen als Verbindungsart die Vernähung oder Verklebung (Laminierung/ Kaschierung) in Frage.

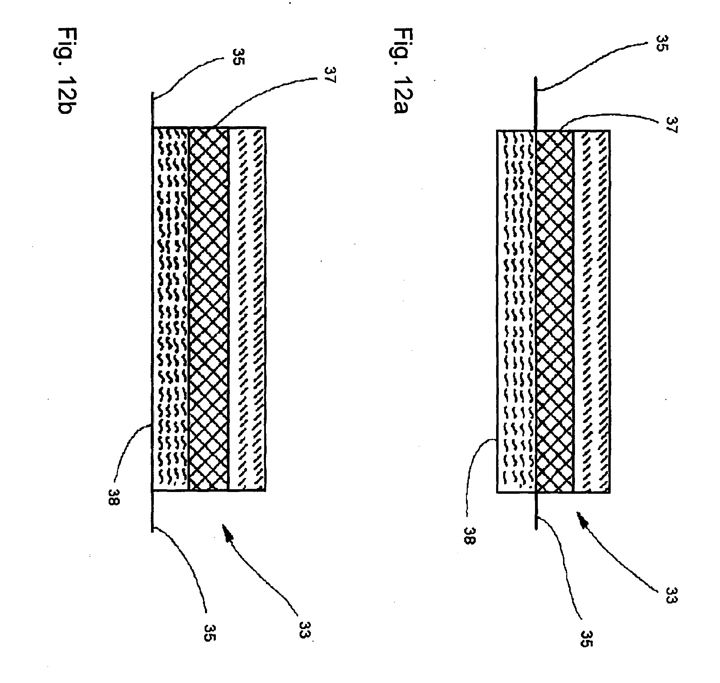

Die Handgriffe 35 können auch, wie durch Zusammenschau mit den in den Fig. 12a bis 12b gezeigten Schnittansichten der Wärmedecke 33 hervorgeht, Bestandteil von in sich geschlossenen Stoff- oder Lederbändern sein. Diese in sich geschlossenen Leder- bzw. Stoffbänder sind, wie die Fig. 12a und 12b zeigen, zwischen Zwischenschicht 37 und Außenlage 38 (Fig. 12a) sowie unterhalb der Außenlage 38 angeordnet. Zudem sind die in sich geschlossenen Leder- und Stoffbänder so ausgeformt, dass die Handgriffe 35 die jeweiligen Enden bilden und die Längsstücke der in sich geschlossenen Leder- und Stoffbänder mit der Zwischenschicht 37 und der Außenlage 38 (Fig. 12a) bzw. nur mit der Außenlage 38 (Fig. 12b) fest verbunden sind. Auch hier kann die feste Verbindung durch Vernähung gegeben sein.

### Bezugszeichenliste

- 100: Rettungswagen
- 9: Leitung
- 10: Patientenraum
- 11: Wand
- 12: Heizungsgitter
- 13: Abdeckplatte
- 13a: Öffnung
- 14: Ausgang
- 14a: Gitter
- 14c: Gitter
- 14d: Steuerung
- 14e: Temperaturregler
- 14f: Temperaturegler
- 15: Fenster
- 16: Verbindungsschlauch
- 17: Anschluss
- 18: Patient
- 19: Wärmedecke
- 20: Trage
- 22: Liegefläche
- 23: Tragenaufnahme
- 24: Ambulanztisch
- 25: Bajonettverschluss
- 26: Leitung
- 26a: Bajonettverschluss
- 27: Verbindungsschlauch
- 27a: Knöpfe
- 28: Dichtlippe
- 28a: Verbindungsmittel
- 30: Feder
- 31: Bimetallfeder
- 31a: Ende
- 32: Vorrichtung
- 32a: Öffnung
- 32b: Kontaktteil
- 35: Handgriff
- 36: Leitung
- 37: Zwischenschicht
- 38: Außenlage

## Patentansprüche

1. Rettungswagen (100) mit einem Ausgang (14) einer Klimaanlage und/oder mit einem Ausgang einer Heizungsanlage zum Kühlen oder Wärmen eines Patientenraumes (10), wobei der Ausgang (14) mit einer eine Öffnung (13a) aufweisenden Abdeckplatte (13) versehen ist und wobei der Öffnung (13a) eine als Rohr oder Schlauch ausgebildete Leitung (9, 26, 36) zur Förderung von Warmluft und/oder Kaltluft zugeordnet ist, wobei die Leitung (9, 26,36) mit einem Verbindungsschlauch (16, 27) verbunden ist und der Verbindungsschlauch (16, 27) an eine Wärmedecke (19) gekoppelt ist, wobei die Wärmedecke (19) als Unterlage vorgesehen ist, so dass sich diese unterhalb eines Patienten befindet, wobei die Wärmedecke (19) mit einer Trage (20) fest oder lösbar verbunden ist.

2. Rettungswagen nach Anspruch 1, **dadurch gekennzeichnet, dass** die Abdeckplatte (13) auf der dem Ausgang (14) zugewandten Seite eine Dichtlippe (28) oder Dichtungsschaumstofffläche aufweist.

3. Rettungswagen nach Anspruch 1 oder 2, **dadurch gekennzeichnet, dass** die Abdeckplatte (13) eine Befestigungsvorrichtung (29) für die Befestigung der Abdeckplatte (13) aufweist.

4. Rettungswagen nach einem der vorhergehenden Ansprüche, **dadurch gekennzeichnet, dass** in der Leitung (9) eine Bimetallfeder (31) angeordnet ist.

5. Rettungswagen nach einem der vorhergehenden Ansprüche, **dadurch gekennzeichnet, dass** die Leitung (9) eine Öffnung (32a) und eine bewegliche Vorrichtung (32) zum Schließen der Öffnung (32a) aufweist.

6. Rettungswagen nach Anspruch 5, **dadurch gekennzeichnet, dass** die Vorrichtung (32) und die Bimetallfeder (31) miteinander gekoppelt sind.

7. Rettungswagen nach einem der vorhergehenden Ansprüche, **dadurch gekennzeichnet, dass** die Leitung (9) mit der Öffnung (13a) der Abdeckplatte (13) gekoppelt ist.

8. Rettungswagen nach Anspruch 7, **dadurch gekennzeichnet, dass** die Leitung (9) über einen Bajonettverschluss an der Öffnung (13a) angeschlossen ist.

9. Rettungswagen nach einem der vorhergehenden Ansprüche, **dadurch gekennzeichnet, dass** die Abdeckplatte (13) des Rettungswagens (100), mit einer Öffnung (13a) versehen ist, wobei der Öffnung (13a) eine Leitung (26, 36) zur Förderung von Warmluft und/oder Kaltluft zugeordnet ist.

10. Rettungswagen nach Anspruch 9, **dadurch gekennzeichnet, dass** die Platte (13) auf einer ihrer Seiten mit einer Dichtlippe (28) oder mit einer Dichtungsschaumstofffläche versehen ist.

11. Rettungswagen nach einem der Ansprüche 9 oder 10, **dadurch gekennzeichnet, dass** die Platte (13) eine Befestigungsvorrichtung (29) für die Befestigung der Platte (13) aufweist.

12. Rettungswagen nach einem der Ansprüche 9 bis 11, **dadurch gekennzeichnet, dass** in der Leitung (9, 26, 36) eine Bimetallfeder (31) angeordnet ist.

13. Rettungswagen nach einem der Ansprüche 9 bis 12, **dadurch gekennzeichnet, dass** die Leitung (9, 26, 36) eine Öffnung (32a) und eine bewegliche Vorrichtung (32) zum Schließen der Öffnung (32a) aufweist.

14. Rettungswagen nach Anspruch 13, **dadurch gekennzeichnet, dass** die Vorrichtung (32) und die Bimetallfeder (31) miteinander gekoppelt sind.

15. Rettungswagen nach einem der Ansprüche 9 bis 14, **dadurch gekennzeichnet, dass** die Leitung (9, 26, 36) mit der Öffnung (32a) gekoppelt ist.

16. Rettungswagen nach Anspruch 15, **dadurch gekennzeichnet, dass** die Leitung über einen Bajonettverschluss (26a) an der Öffnung (13a) angeschlossen ist.

17. Rettungswagen nach einem der Ansprüche 9 bis 16, **dadurch gekennzeichnet, dass** die Leitung (9, 26, 36) über einen Verbindungsschlauch an eine Wärmedecke gekoppelt ist.

## Claims

1. An ambulance (100) with an outlet (14) of an air-conditioning system and/or with an outlet of a heating system for cooling or warming a patient compartment (10), wherein the outlet (14) is provided with a cover plate (13) having an opening (13a) and wherein the opening (13a) has assigned to it a pipeline (9, 26, 36), designed as a pipe or hose, for conveying warm air and/or cold air, wherein the pipeline (9, 26, 36) is connected to a connection hose (16, 27) and the connection hose (16, 27) is coupled to a heat blanket (19), wherein the heat blanket (19) is envisaged as a substrate so that it is located underneath a patient, wherein the heat blanket (19) is firmly or detachably connected to a stretcher (20).

2. The ambulance according to claim 1, **characterised in that** on the side facing the outlet (14), the cover plate (13) has a sealing lip (28) or sealing foam area.

3. The ambulance according to claim 1 or 2, **characterised in that** the cover plate (13) has a fastening device (29) for fastening the cover plate (13).

4. The ambulance according to any one of preceding claims, **characterised in that** a bimetallic spring (31) is arranged in the pipeline (9).

5. The ambulance according to any one of the preceding claims, **characterised in that** the pipeline (9) has an opening (32a) and a movable device (32) for closing the opening (32a).

6. The ambulance according to claim 5, **characterised in that** the device (32) and bimetallic spring (31) are coupled to each other.

7. The ambulance according to any one of the preceding claims, **characterised in that** the pipeline (9) is coupled to the opening (13a) of the cover plate (13).

8. The ambulance according to claim 7, **characterised in that** the pipeline (9) is connected by means of a bayonet fastening to the opening (13a).

9. The ambulance according to any one of the preceding claims, **characterised in that** cover plate (13) of the ambulance (100) is provided with an opening (13a), wherein allocated to the opening (13a) is a pipeline (26, 36) for conveying warm air and/or cold air.

10. The ambulance according to claim 9, **characterised in that** on one of its sides the plate (13) is provided with a sealing lip (28) or with a sealing foam area.

11. The ambulance according to one of claims 9 or 10, **characterised in that** the plate (13) has a fastening device (29) for fastening the plate (13).

12. The ambulance according to any one of claims 9 to 11, **characterised in that** a bimetallic spring (31) is arranged in the pipeline (9, 26, 36).

13. The ambulance according to any one of claims 9 to 12, **characterised in that** the pipeline (9, 26, 36) has an opening (32a) and a moveable device (32) for closing the opening (32a).

14. The ambulance according to claim 13, **characterised in that** the device (32) and the bimetallic spring (31) are coupled to each other.

15. The ambulance according to any one of claims 9 to 14, **characterised in that** pipeline (9, 26, 36) is connected with the opening (32a).

16. The ambulance according to claim 15, **characterised in that** pipeline is connected to the opening (13a) by means of a bayonet fastening (26a).

17. The ambulance according to any one of claims 9 to 16, **characterised in that** pipeline (9, 26, 36) is connected to a heat blanket by way of a connection hose.

## Revendications

1. Ambulance (100) avec une sortie (14) d'une climatisation et/ou avec une sortie d'un système de chauffage pour refroidir ou pour chauffer un espace dédié au patient (10), la sortie (14) étant munie d'une plaque de recouvrement (13) comportant un orifice (13a) et à l'orifice (13a) étant associé un conduit (9, 26, 36) conçu en tant que tuyau ou en tant que flexible, pour le transport d'air chaud et/ou d'air froid, le conduit (9, 26, 36) étant relié à un flexible de liaison (16, 27) et le flexible de liaison (16, 27) étant couplé à une couverture chauffante (19), la couverture chauffante (19) étant prévue en tant que couche de base, de sorte qu'elle se trouve en-dessous d'un patient, la couverture chauffante (19) étant reliée de manière fixe ou amovible avec un brancard (20).

2. Ambulance selon la revendication 1, **caractérisée en ce que** sur le côté faisant face à la sortie (14), la plaque de recouvrement (13) comporte une lèvre d'étanchéité (28) ou une surface en mousse d'étanchéité.

3. Ambulance selon la revendication 1 ou 2, **caractérisée en ce que** la plaque de recouvrement (13) comporte un dispositif de fixation (29) pour la fixation de la plaque de recouvrement (13).

4. Ambulance selon l'une quelconque des revendications précédentes, **caractérisée en ce que** dans le conduit (9) est placé un ressort (31) bimétallique.

5. Ambulance selon l'une quelconque des revendications précédentes, **caractérisée en ce que** le conduit (9) comporte un orifice (32a) et un dispositif (32) mobile pour fermer l'orifice (32a).

6. Ambulance selon la revendication 5, **caractérisée en ce que** le dispositif (32) et le ressort (31) bimétallique sont couplés l'un à l'autre.

7. Ambulance selon l'une quelconque des revendications précédentes, **caractérisée en ce que** le conduit (9) est couplé avec l'orifice (13a) de la plaque de recouvrement (13).

8. Ambulance selon la revendication 7, **caractérisée en ce que** le conduit (9) est raccordé sur l'orifice (13a) par l'intermédiaire d'une fermeture à baïonnette.

9. Ambulance selon l'une quelconque des revendications précédentes, **caractérisée en ce que** la plaque de recouvrement (13) de l'ambulance (100) est munie d'un orifice (13a), à l'orifice (13a) étant associé un conduit (26, 36) pour le transport d'air chaud et/ou d'air froid.

10. Ambulance selon la revendication 9, **caractérisée en ce que** sur l'une de ses faces, la plaque (13) est munie d'une lèvre d'étanchéité (28) ou d'une surface en mousse d'étanchéité.

11. Ambulance selon l'une quelconque des revendications 9 ou 10, **caractérisée en ce que** la plaque (13) comporte un dispositif de fixation (29) pour la fixation de la plaque (13).

12. Ambulance selon l'une quelconque des revendications 9 à 11, **caractérisée en ce que** dans le conduit (9, 26, 36) est placé un ressort (31) bimétallique.

13. Ambulance selon l'une quelconque des revendications 9 à 12, caractérisée que le conduit (9, 26, 36) comporte un orifice (32a) et un dispositif (32) mobile pour fermer l'orifice (32a).

14. Ambulance selon la revendication 13, **caractérisée en ce que** le dispositif (32) et le ressort (31) bimétallique sont couplés l'un à l'autre.

15. Ambulance selon l'une quelconque des revendications 9 à 14, **caractérisée en ce que** le conduit (9, 26, 36) est couplé à l'orifice (32a).

16. Ambulance selon la revendication 15, **caractérisée en ce que** le conduit est raccordé sur l'orifice (13a) par l'intermédiaire d'une fermeture à baïonnette (26a).

17. Ambulance selon l'une quelconque des revendications 9 à 16, **caractérisée en ce que** le conduit (9, 26, 36) est couplé à une couverture chauffante par l'intermédiaire d'un flexible de liaison.
